# EUROPEAN PATENT APPLICATION

(11) **EP 4 174 553 A1**
(43) Date of publication of application: **03.05.2023**
(21) Application number: 21205292.2
(22) Date of filing: 28.10.2021
(51) Int. Cl.: G02B 21/16, G02B 21/00, G06T 7/00

(54) **SYSTEM, METHOD AND COMPUTER PROGRAM FOR A MICROSCOPE OF A SURGICAL MICROSCOPE SYSTEM**

(71) Applicant: Leica Instruments (Singapore) Pte. Ltd., Singapore 608924 (SG)
(72) Inventor: THEMELIS, George, 88131 Lindau (DE)
(74) Representative: 2SPL Patentanwälte PartG mbB

(57) **Abstract**

Examples relate to a system (110), a method and a computer program for a microscope (120) of a surgical microscope system (100), to a corresponding surgical microscope system comprising such a system. The system (110) is configured to obtain imaging sensor data from at least one optical imaging sensor (122) of the microscope. The imaging sensor data comprises a first component that is based on reflectance imaging and a second component that is based on fluorescence imaging. The system is configured to generate a digital view of a surgical site based on at least the first component of the imaging sensor data. The system is configured to detect at least one feature of interest in the second component of the imaging sensor data. The system is configured to generate a visual indicator of the at least one feature of interest. The system is configured to provide a display signal to a display device (130) of the surgical microscope system, the display signal comprising the digital view and the visual indicator.

## Description

### Technical field

Examples relate to a system, a method, and a computer program for a microscope of a surgical microscope system and to a corresponding surgical microscope system comprising such a system.

### Background

In research and development, surgical microscopes are being developed that collect multiple images at a time, in addition to further data, which is then made available to the surgeon. For example, fluorescence imaging overlays being overlaid over a reflectance images allow the surgeon to perceive, in a single view, at least two different images: white light color and fluorescence. This offers to the surgeon continuous awareness of both imaging modes. However, the more images and data are collected, the more difficulties arise in making the wealth of information available to the surgeon without causing overload.

In the absence of a fluorescence (FL) signal, the combined fluorescence and white light image may look identical or similar to the white light (WL) image. Therefore, it can be difficult for the surgeon to know if they are working in the white light or combined fluorescence and white light view, which may lead to a lack of information for the surgeon. On the other hand, when multiple imaging modes are available, it may be impractical or even impossible to visualize everything in a single image. Moreover, information overload can be counterproductive.

### Summary

There may be a desire to provide an improve concept for collecting and visualizing images and data of a surgical site.

This desire is addressed by the subject-matter of the independent claims.

Various examples of the present disclosure are based on the finding, that fluorescence imaging is particularly suitable for detecting and identifying features of interest to be shown in a digital view of a surgical microscope system. Moreover, oftentimes, due to the low luminance of the fluorescence emissions, the small size of the sources of the fluorescence emissions, and the inherent wealth of information shown in the digital view, the fluorescence emissions may be hard to perceive in a digital view, to the extent, that, when the fluorescence emissions do not stand out, the surgeon may have a hard time noticing whether the digital view only shows reflectance imaging based images (i.e., white light images) of combined reflectance imaging and fluorescence imaging based images. In the proposed concept, imaging sensor data is collected that comprises a first component that is based on reflectance imaging and a second component that is based on fluorescence imaging. The second, fluorescence imaging-based component is analyzed to detect features of interest. If such a feature of interest is detected, a visual indicator, such as an outline, an arrow etc. is generated and included in the digital view of the surgical site.

Various examples of the present disclosure relate to a system for a microscope of a surgical microscope system. The system comprises one or more processors and one or more storage devices. The system is configured to obtain imaging sensor data from at least one optical imaging sensor of the microscope. The imaging sensor data comprises a first component that is based on reflectance imaging and a second component that is based on fluorescence imaging. The system is configured to generate a digital view of a surgical site based on at least the first component of the imaging sensor data. The system is configured to detect at least one feature of interest in the second component of the imaging sensor data. The system is configured to generate a visual indicator of the at least one feature of interest. The system is configured to provide a display signal to a display device of the surgical microscope system, the display signal comprising the digital view and the visual indicator. By detecting the feature of interest in the fluorescence imaging based data, the occurrence of a feature of interest that may or may not be apparent to the surgeon can be detected. By including a visual indicator, the surgeon may be made aware of the feature of interest, in particular when it is otherwise imperceptible or hard to perceive in the digital view.

For example, the system may be configured to detect the at least one feature of interest based on fluorescence emissions emitted by the feature of interest. In other words, the fluorescence emissions, which are represented by the second component, may be analyzed to detect the feature of interest.

In some cases, such a visual indicator might not be necessary, e.g., when the feature of interest is clearly shown in the digital view, e.g., in a pseudocolor representation. However, as outlined above, the digital view can also be generated without generating such a fluorescence imaging overlay, as a white light-only image. In this case, the indicator may be shown to make the surgeon aware of the feature of interest. For example, the system may be configured to generate the visual indicator on the at least one feature of interest if the second component is omitted in the generation of the digital view.

Other cases may not be as clear cut as the case outlined above. For example, in some cases, the fluorescence imaging overlay is included in the digital view, but the low luminance or small size of patches of tissue emitting the fluorescence emissions make them hard to perceive in the digital view. The system may be configured to determine a visibility of the at least one feature of interest, and to generate a visual indicator for a feature of interest if the visibility of the feature of interest is deemed to fail a visibility criterion. By determining the visibility, the visual indicator may be shown in scenarios where the feature of interest may be hard to perceive.

For example, a feature of interest detected in the second component of the imaging sensor data may be deemed to fail the visibility criterion if the second component is omitted in the generation of the digital view. In other words, if the fluorescence imaging component is not represented in the digital view, the feature of interest may be deemed not to be sufficiently visible.

In some examples the system is configured to generate the digital view based on the first component and based on the second component of the imaging sensor data. For example, a pseudocolor representation of the second component may be overlaid over the color representation of the first component in the digital view.

If both the first and the second component are represented in the digital view, the second component may still be hard to perceive, in particular if the patches of tissue that emit fluorescence emissions are small. Accordingly, the system may be configured to determine a feature detected in the second component to fail the visibility criterion if the size of the feature, relative to the digital view, is below a size threshold.

Another challenge lies in the complexity of the white light images of the surgical site, i.e., the first component, where the tissue may exhibit many colors and color transitions, shapes, and structures. In some cases, the fluorescence overlay may be hard to perceive, e.g., due to low contrast to the adjacent tissue or as the low intensity of the fluorescence emission result in a low intensity of the overlay. The system may be configured to determine a feature detected in the second component to fail the visibility criterion if a contrast or intensity of fluorescence imaging indicator highlighting the feature in the digital view relative to adjacent portions of the digital view is below a contrast threshold or intensity threshold, respectively.

To sum up, various criteria may be used to define the at least one visibility criterion. For example, the at least one visibility criterion may be based on at least one of a size of the one or more features of interest, a contrast of the one or more features of interest relative to the digital view, a visual intensity of the one or more features of interest in the digital view, and the presence of an indicator highlighting the one or more features of interest in the digital view.

As outlined above, the digital view may represent both the first component (that is based on reflectance imaging) and the second component (that is based on fluorescence imaging). For example, the system may be configured to generate the digital view as a composite image with a pseudocolor representation of the second component that is combined with a color representation of the first component or as an image with a color representation of the first component and a monochrome representation of the second component that are shown separately. In other words, if both the first and second component are represented, they may be shown overlaid over each other, or side by side.

In some examples, the detection of the feature of interest may be based on a simple algorithm that determines the size and/or intensity of the fluorescence emissions. If at least one of the size and the intensity reaches a respective threshold, the respective emissions may be deemed to be a feature of interest. In some examples, however, more sophisticated techniques may be used. For example, object detection, which may include object classification, may be used to detect the at least one feature of interest. For example, the system may be configured to perform object detection based on the first and/or second component of the optical imaging data to determine whether a feature is a feature of interest.

Various types of visual indicators may be used to highlight the feature of interest. For example, the visual indicator may be one of a blinking indicator, an indicator having an increased contrast relative to adjacent portions of the digital view, an indicator drawing an outline around the feature of interest, and an indicator for prompting a surgeon to switch the digital view to show fluorescence emissions in isolation. The system may be configured to include the visual indicator in the display signal as one of an overlay, an indicator included in a composite reflectance and fluorescence image, and a warning image.

Various examples of the present disclosure relate to a surgical microscope system comprising the system introduced above and the microscope.

Various examples of the present disclosure relate to a corresponding method for a microscope of a surgical microscope system. The method comprises obtaining imaging sensor data from at least one optical imaging sensor of the microscope. The imaging sensor data comprises a first component that is based on reflectance imaging and a second component that is based on fluorescence imaging. The method comprises generating a digital view of a surgical site based on at least the first component of the imaging sensor data. The method comprises detecting at least one feature of interest in the second component of the imaging sensor data. The method comprises generating a visual indicator of the at least one feature of interest. The method comprises providing a display signal to a display device of the surgical microscope system, the display signal comprising the digital view and the visual indicator.

Various examples of the present disclosure relate to a computer program with a program code for performing the above method when the computer program is executed on a processor.

### Short description of the Figures

Some examples of apparatuses and/or methods will be described in the following by way of example only, and with reference to the accompanying figures, in which
- Fig. 1a: shows a block diagram of an example of a system for a microscope of a surgical microscope system;
- Fig. 1b: shows a schematic diagram of an example of a surgical microscope system;
- Fig. 2: shows a flow chart of an example of a method for a microscope of a surgical microscope system;
- Fig. 3a: shows a schematic drawing of an example of a combined white light and fluorescence digital view;
- Fig. 3b: shows a schematic diagram of an example of a fluorescence digital view; and
- Fig. 4: shows a schematic diagram of an example of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

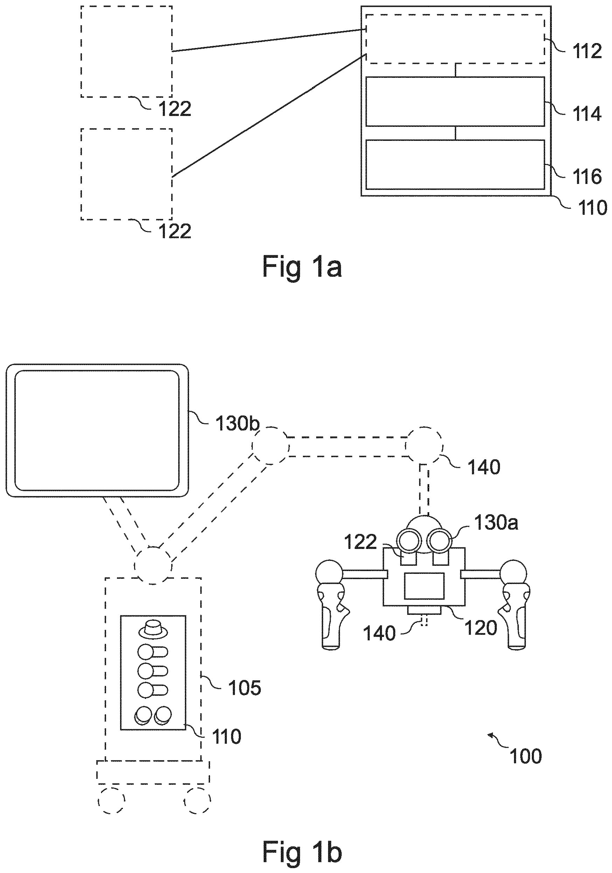

Fig. 1a shows a block diagram of an example of a system 110 for a microscope 120 of a surgical microscope system 100. The system comprises one or more processors 114 and one or more storage devices 116. Optionally, the system further comprises one or more interfaces 112. The one or more processors 114 are coupled to the one or more storage devices 116 and to the optional one or more interfaces 112. In general, the functionality of the system is provided by the one or more processors, in conjunction with the one or more interfaces (for exchanging information, e.g., with an optical imaging sensor of a microscope and/or with a display device of the surgical microscope system) and/or with the one or more storage devices (for storing and/or retrieving information).

The system is configured to obtain imaging sensor data from at least one optical imaging sensor 122 of the microscope. The imaging sensor data comprises a first component that is based on reflectance imaging and a second component that is based on fluorescence imaging. The system is configured to generate a digital view of a surgical site based on at least the first component of the imaging sensor data. The system is configured to detect at least one feature of interest in the second component of the imaging sensor data. The system is configured to generate a visual indicator of the at least one feature of interest. The system is configured to provide a display signal to a display device 130 of the surgical microscope system, the display signal comprising the digital view and the visual indicator.

The proposed concept, and in particular the proposed system 110, is a system for a microscope of a surgical microscope system. For example, the system 110 may be a computer system that is coupled with the microscope 120 (as shown in Fig. 1b). The system 110 may provide processing capabilities and/or control capabilities that may be used to augment the functionality of the surgical microscope system, and in particular of the microscope. For example, the proposed system may assist a user of the microscope, e.g., a surgeon, by providing the digital view with the visual indicator, thereby highlighting features of interest that may be relevant to the surgeon.

The proposed system is used in conjunction with the microscope 120. In general, a microscope is an optical instrument that is suitable for examining objects that are too small to be examined by the human eye (alone). For example, a microscope may provide an optical magnification of a sample. In modern microscopes, the optical magnification is often provided for a camera or an imaging sensor, such as the optical imaging sensor 122 of the microscope 120 that is shown in Fig. 1b. The microscope 120 may further comprise one or more optical magnification components that are used to magnify a view on the sample, such as an objective (i.e., lens).

There are a variety of different types of microscopes. If the microscope is used in the medical or biological fields, the object being viewed through the microscope may be a sample of organic tissue, e.g., arranged within a petri dish or present in a part of a body of a patient. In the present case, the microscope is a microscope of a surgical microscope system, i.e., a microscope that is to be used during a surgical procedure, such as an oncological surgical procedure or during tumor surgery. Such a system is shown in Fig. 1b, for example. Accordingly, an object being viewed through the microscope, and shown in the image data, may be a sample of organic tissue of a patient, and may be in particular be a surgical site that the surgeon operates on during the surgical procedure.

The above system 110 is suitable for use with the surgical microscope system comprising the microscope 120, e.g., as part of the surgical microscope system 100. Fig. 1b shows a schematic diagram of an example of the surgical microscope system 100 comprising the system 110 and the microscope 120. The microscope system shown in Fig. 1b is a surgical microscope system. However, the system 110 may be used with other microscope systems or optical systems as well. The surgical microscope system 100 shown in Fig. 1b comprises a number of optional components, such as a base unit 105 (comprising the system 110) with a (rolling) stand, ocular displays 130a that are arranged at the microscope 120, an auxiliary display 130b that is arranged at the base unit and a (robotic or manual) arm 140 which holds the microscope 120 in place, and which is coupled to the base unit 105 and to the microscope 120. In the context of this application, the term "(surgical) microscope system" is used, in order to cover the portions of the system that are not part of the actual microscope (which comprises the optical components), but which are used in conjunction with the microscope, such as the display or an illumination system.

The proposed concept focuses on tasks being performed by the system 110. The system 110 is tasked with providing a display signal with a digital view of the surgical site. As outlined above, in modern surgical microscopes, the view on the surgical site is often provided as a digital view. The microscope comprises at least one optical imaging sensor that is used to generate imaging sensor data of the surgical site. This imaging sensor data is processed by the system 110, and a digital view is generated of the surgical site based on the processed imaging sensor data. For example, the digital view may be based on the field of view of the microscope, as sensed by the optical imaging sensor.

In the present disclosure, the imaging sensor data comprises two components - the first component, which is based on reflectance imaging, and the second component, which is based on fluorescence imaging. Accordingly, the surgical microscope system is configured to perform reflectance imaging and fluorescence imaging. In reflectance imaging, light is emitted, by an illumination system of the surgical microscope system, towards the surgical site, and reflected by the surgical site, e.g., towards the optical imaging sensor. The optical imaging sensor thus senses the light that is reflected by the surgical site, with the light being sensed being in the same wavelength band as the light being emitted towards the surgical site.

In contrast, in fluorescence imaging, light is emitted towards the surgical site in a first wavelength band (also called the fluorescence excitation wavelength band). If a fluorescent dye is used in the surgical site, the emitted light excites the dye, and light is emitted in a second wavelength band (also called the fluorescence emission wavelength band). This light can be recorded by a camera sensor, such as the optical imaging sensor, and displayed on a display of the surgical microscope. Compared to reflectance imaging, in reflectance imaging, light that is emitted by the fluorescent dye has a low illumination intensity, and thus cannot be distinguished without digital processing if the surgical site is illuminated for reflectance imaging.

To enable concurrent reflectance imaging and fluorescent imaging, the fluorescence emission wavelength band (or fluorescence emission wavelength bands, if multiple fluorescent dyes having different fluorescence emission wavelength bands are used) may be sensed separately by the at least one optical imaging sensor, and light in the fluorescence emission wavelength band(s) (and optionally in the fluorescence excitation wavelength band(s)) may be omitted or filtered out from the illumination being used for reflectance imaging. In some examples, different optical imaging sensors are used for fluorescence imaging and reflectance imaging. For example, the microscope may comprise a first optical imaging sensor being configured to perform reflectance imaging and a second optical imaging sensor being configured to perform fluorescence imaging. The system may be configured to obtain the first component from the first optical imaging sensor being configured to perform reflectance imaging and to obtain the second component from the second optical imaging sensor being configured to perform fluorescence imaging. In some examples, wavelength bands are recorded separately by one or more optical imaging sensors (e.g., for the purposes of multispectral images, e.g., using filters that are placed in front of the optical imaging sensors), and one or more of the wavelength bands may be used for fluorescence imaging, e.g. by blocking light from the emission spectrum of the illumination system of the surgical microscope system that intersects with the fluorescence emission wavelength band.

The system is configured to obtain the imaging sensor data from the at least one optical imaging sensor 122 of the microscope, with the imaging sensor data comprising a first component that is based on reflectance imaging and a second component that is based on fluorescence imaging. For example, at least part of the time, i.e., when reflectance imaging and fluorescence imaging is used at the same time, the two components may (both) be present in the imaging sensor data at the same time. For example, the imaging sensor data may comprise first imaging sensor data from a first optical imaging sensor being used for reflectance imaging and second imaging sensor data from a second optical imaging sensor being used for fluorescence imaging. The first imaging sensor data may correspond to or comprise the first component, and the second imaging sensor data may correspond to or comprise the second component. Alternatively, a single optical imaging sensor data may be used, in combination with one or more bandpass filters that are arranged in front of the pixels of the optical imaging sensor. Imaging sensor data of pixels that admit light in the fluorescence emission wavelength band (due to the light not being filtered out by one of the one or more bandpass filters) may correspond to or comprise the second component. Imaging sensor data of the remaining pixels, or imaging sensor data of all pixels, may correspond to or comprise the first component. The system may be configured to separate the first and second component based on the imaging sensor data, e.g., on a pixel-by-pixel basis, or based on the imaging sensor data from different optical imaging sensors.

In general, the reflectance imaging may be used to generate a color image of the surgical site, while the fluorescence imaging may be used to generate an image of the surgical site that represents the intensity of the fluorescence emissions, e.g., as monochrome image. The digital view may be generated based on these images.

At the very least, the digital view is generated based on the first component. For example, the system may be configured to generate the digital view with a first representation comprising or corresponding to a color representation of the first component, thereby providing a reflectance image or "white light" image of the surgical site. In the first representation, the second component is omitted. In other words, the second component may be omitted in the generation of the digital view if (only) the first representation is shown (without showing a second representation as presented in the following).

In addition, the system may be configured to generate the digital view based on the second component. In other words, the system may be configured to generate the digital view based on the first component and based on the second component of the imaging sensor data. For example, the system may be configured to generate the digital view with a second representation comprising or corresponding to a monochrome representation of the second component, thereby providing a fluorescence image of the surgical site. For example, a gradient between black and white may be used to represent the intensity of the fluorescence emissions. For example, the system may be configured to switch between the first representation and the second representation upon request by the surgeon, or to show the first representation and the second representation side by side or in a picture-in-picture arrangement. In other words, the system may be configured to generate the digital view as an image with a color representation of the first component and a monochrome representation of the second component that are shown separately, e.g., side by side or in a picture-in-picture arrangement.

A third option is to use a combined representation. For example, the system may be configured to generate the digital view with a third representation comprising a color representation of the first component and a (monochromatic) pseudocolor representation of the second component. In other words, the system may be configured to generate the digital view as a composite image with a pseudocolor representation of the second component that is combined with a color representation of the first component. For example, the pseudocolor representation of the second component may comprise a single color (i.e., be monochromatic), with different intensities (or transparency values) being used to represent the intensity of the fluorescence emission. For example, a highly visible color may be used for the pseudocolor representation, e.g., a color that is not usually found in surgical sites (e.g., (bright) blue, (bright) green or (bright) pink, e.g., neon blue, neon green or neon pink). The pseudocolor representation may be overlaid, i.e., superimposed, over the color representation of the first component. For example, the system may be configured to switch between the first representation, the second representation and the third representation upon request by the surgeon. In the present disclosure, the first representation is also denoted white light image, the second representation is also denoted fluorescence image, and the third representation is also denoted combined fluorescence and white light image.

The proposed concept is based on the insight, that, when such a digital view with the first and/or third representation is generated, some features that are possibly of interest to the surgeon are either not shown at all (if the first representation is used and the feature of interest is only visible in the fluorescence image) or are hard to perceive in the combined view (i.e., the third representation, e.g., as the contrast or size of the feature makes it hard to see). Therefore, the system is configured to detect the least one feature of interest in the second component of the imaging sensor data. In this context, the feature of interest may be a portion of tissue that emits fluorescence emissions. For example, the feature of interest may be a portion of tissue emitting fluorescence emissions, with the portion of tissue being larger that a pre-defined size, or with the intensity of the fluorescence emissions being higher than an intensity threshold. For example, the feature of interest may be a portion of tissue to be removed by the surgeon, e.g., a tumor, that is infused with a fluorescent dye. Accordingly, the system may be configured to detect the at least one feature of interest based on fluorescence emissions emitted by the feature of interest.

In addition to, or as an alternative to, the size and intensity of the fluorescence emissions, the shape of the fluorescence emissions may be of interest, too. For example, fluorescent dyes may be used to clearly identify aneurysms in vascular surgery. However, in this case, the aneurysm might not emit fluorescence emissions, in contrast to neighboring vessels. Therefore, in this case, the feature of interest, i.e., the aneurysm, may be detected due to a lack of fluorescence emissions compared to neighboring vessels or portions of the same vessel. For example, the system may be configured to use object detection to identify the shape of a blood vessel or network of blood vessels, and to detect a feature of interest if a portion of the blood vessel or network of blood vessels emits less (or none) fluorescence emissions compared to adjacent portions of the blood vessel or network of blood vessels. For example, the object detection may be applied on the second component, on the first component, or on a combination of the first and second component. In a more general manner, object detection may be used to distinguish features from features of interest. For example, the system may be configured to perform object detection based on the first and/or second component of the optical imaging data to determine whether a feature is a feature of interest. For example, the object detection may be based on image segmentation and image classification. For example, a machine-learning model may be trained to perform image segmentation and classification on the first component, e.g., to segment the first component into smaller image portions (image segmentation), with each image portion showing a feature, and with the image classification being used to classify the features shown in the smaller image portions, e.g., into different types of objects. Depending on the classification, a feature may be deemed to be a potential feature of interest. Based on whether the potential feature of interest also emits fluorescence emissions (i.e., based on the second component), it may be deemed to be a feature of interest.

The training of machine-learning models to perform image segmentation and classification is well-known in the art. For example, a supervised learning-based approach may be used, where annotated images are used as desired training output and the same images are used, without annotations, as training input.

If a feature of interest is detected, a visual indicator of the at least one feature of interest may be generated. However, not every feature of interest being detected might lead to a visual indicator being generated. In some examples, the feature of interest might be easy to spot in the digital view (as it is currently being generated), so the generation of a corresponding visual indicator may be omitted. This is primarily the case if the combined view, i.e., the third representation, or both the first and the second representation, are shown in the digital view of the surgical site. However, if the digital view shows the first representation (without showing the second or third representation as well), features that are only or primarily perceivable or distinguishable due to their fluorescence emissions (or lack thereof) may be overlooked due to lack of visibility. For example, the system may be configured to determine a visibility of the at least one feature of interest, and to generate a visual indicator for a feature of interest if the visibility of the feature of interest is deemed to fail a visibility criterion. A feature of interest detected in the second component of the imaging sensor data is deemed to fail the visibility criterion if the second component is omitted in the generation of the digital view (e.g., when the first representation is shown in isolation). Therefore, the system may be configured to generate the visual indicator on the at least one feature of interest if the second component is omitted in the generation of the digital view (and omit generating the visual indicator if the second component is also represented in the digital view, e.g., if the second or third representation are shown).

In some cases, the visibility of the feature of interest may also be impacted if the digital view comprises the third representation, e.g., if the pseudocolor representation has low contrast to adjacent portions of the digital view, if the intensity of the pseudocolor representation is low due to a low intensity of the fluorescence emissions, or if the size of the feature of interest is (very) small. Accordingly, the system may be configured to determine a feature detected in the second component to fail the visibility criterion if the size of the feature, relative to the digital view, is below a size threshold. In other words, the size (e.g., the area) of the feature of interest may be compared to the overall size (e.g., area) of the digital view (e.g., at the current level of magnification). If the size (e.g., area) of the feature of interest is below the size threshold, e.g., less than 5% (or less than 2%, or less than 1%) of the area of the digital view, the feature of interest may be deemed to fail the visibility criterion.

Another potential visibility criteria are the contrast and the intensity of the representation of the feature of interest. As outlined above, in the combined view (i.e., the third representation), a pseudocolor representation of the fluorescence emissions is overlaid over the color image of the reflectance image. While the color of the pseudocolor representation is chosen to provide high contrast in usual surgical settings, if the intensity of the fluorescence emissions, and thus the intensity of the pseudocolor representation, is low, and/or the color of adjacent portions of the color representation of the first component is similar, the visibility of the feature of interest may be impacted. Accordingly, the system may be configured to determine a feature detected in the second component to fail the visibility criterion if a contrast or intensity of fluorescence imaging indicator highlighting the feature in the digital view relative to adjacent portions of the digital view is below a contrast threshold or intensity threshold, respectively. For example, the fluorescence imaging indicator may correspond to the pseudocolor representation of the second component, at the location of the feature of interest. The contrast may be determined by comparing the color values (e.g., the Red-Green-Blue (RGB) color values) of the pixels of the pseudocolor representation with the color values (e.g., RGB color values) of pixels of the color representation that are adjacent to the pixels of the pseudocolor representation. If the difference in color values is smaller than a threshold (in all color channels), the contrast may be deemed to fail the contrast threshold. Similarly, if the intensity of the fluorescence emissions, and thus also of the pseudocolor representation of the fluorescence emissions, is below the intensity threshold, the intensity of the pseudocolor representation (i.e., the fluorescence imaging indicator) may be deemed to fail the intensity threshold. In summary, the at least one visibility criterion may be based on at least one of a size of the one or more features of interest, a contrast of the one or more features of interest relative to the digital view (e.g., the contrast of the pseudocolor representation of the fluorescence emissions of the feature of interest relative to the adjacent color representation of the surgical site), a visual intensity of the one or more features of interest in the digital view, and the presence of an indicator highlighting the one or more features of interest in the digital view (i.e., is the pseudocolor representation shown or not, i.e., is the third or first representation shown in the digital view).

The purpose of the visual indicator is to make the surgeon aware of the feature of interest, e.g., by highlighting the feature of interest, by delineating the feature of interest from adjacent portions of the digital view, or by making the surgeon aware that the feature of interest can be perceived by switching the digital view, e.g., to the second or third representation. For the first approach of highlighting the feature of interest, a blinking visual indicator, an indicator having an increased contrast, or an indicator drawing an outline around the feature of interest may be used. For the second approach of delineating the feature of interest, the indicator having an increased contrast, or the indicator drawing an outline around the feature of interest may be used. For the third approach of making the surgeon aware that the feature of interest can be perceived by switching the digital view, an indicator, such as a textual indicator or symbol, for prompting a surgeon to switch the digital view may be used. In summary, the visual indicator may be one of a blinking indicator, an indicator having an increased contrast relative to adjacent portions of the digital view, an indicator drawing an outline around the feature of interest, and an indicator for prompting a surgeon to switch the digital view to show fluorescence emissions in isolation. It may be superimposed on the digital view (e.g., when using an outline or a blinking indicator), integrated as part of the digital view (e.g., when increasing the contrast of a portion of the digital view), or be shown next to the digital view (as prompt for switching the digital view). Accordingly, the system may be configured to include the visual indicator in the display signal as one of an overlay (e.g., by superimposing the visual indicator on the digital view), an indicator included in a composite reflectance and fluorescence image (i.e., the third representation, e.g., by altering the composition of the composite reflectance and fluorescence image), and a warning image (to be shown superimposed on the digital view or next to the digital view).

The display signal is provided to a display 130a; 130b of the microscope system 100. For example, the display signal may be a signal for driving (e.g., controlling) the display 130a; 130b. For example, the display signal may comprise video data and/or control instructions for driving the display. For example, the display signal may be provided via one of the one or more interfaces 112 of the system. Accordingly, the system 110 may comprise a video interface 112 that is suitable for providing the video signal to the display of the touch screen. The display signal comprises the digital view and the visual indicator. In other words, the visual indicator and the digital view are combined, as outlined above, and provided within the display signal.

In the proposed concept, the optical imaging sensor of the microscope is employed by the system to generate imaging sensor data of the surgical site, which the system may in turn evaluate to generate the digital view and to detect the at least one feature of interest. Accordingly, the optical imaging sensor 122 is configured to generate the imaging sensor data. For example, the optical imaging sensor of the microscope 120 may comprise or be an APS (Active Pixel Sensor) - or a CCD (Charge-Coupled-Device)-based imaging sensor 122. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging. The system 110 may be configured to obtain (i.e., receive or read out) the imaging sensor data from the optical imaging sensor. The imaging sensor data may be obtained by receiving the imaging sensor data from the optical imaging sensor (e.g., via the interface 112), by reading the imaging sensor data out from a memory of the optical imaging sensor (e.g., via the interface 112), or by reading the imaging sensor data from a storage device 116 of the system 110, e.g., after the imaging sensor data has been written to the storage device 116 by the optical imaging sensor or by another system or processor.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

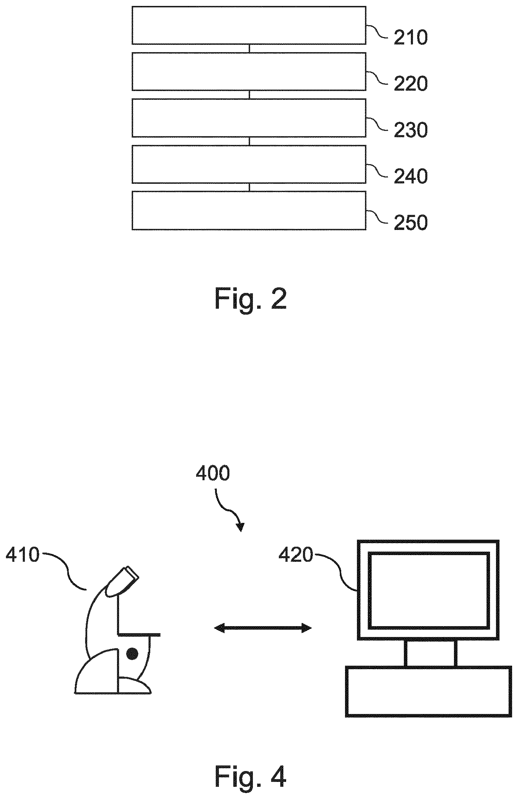

Fig. 2 shows a flow chart of an example of a corresponding method for a microscope of a surgical microscope system, e.g., for the microscope 120 of the surgical microscope system 100 of Figs. 1a and 1b. For example, the method may be performed by the surgical microscope system 100 of Figs. 1a and/or 1b, and in particular by the system 110 of Figs. 1a and/or 1b. The method comprises obtaining 210 imaging sensor data from at least one optical imaging sensor of the microscope. The imaging sensor data comprises a first component that is based on reflectance imaging and a second component that is based on fluorescence imaging. The method comprises generating 220 a digital view of a surgical site based on at least the first component of the imaging sensor data. The method comprises detecting 230 at least one feature of interest in the second component of the imaging sensor data. The method comprises generating 240 a visual indicator of the at least one feature of interest. The method comprises providing 250 a display signal to a display device of the surgical microscope system, the display signal comprising the digital view and the visual indicator.

Features described in connection with the system 110 and/or the microscope system 100 of Fig. 1b may be likewise applied to the method of Fig. 2.

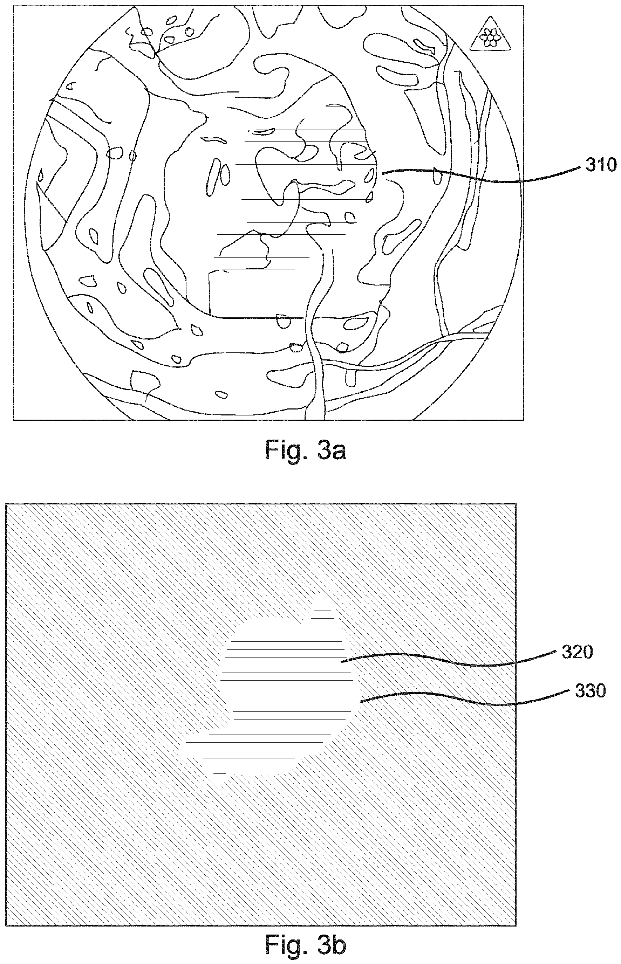

More details and aspects of the method are mentioned in connection with the proposed concept or one or more examples described above or below (e.g. Fig. 1a to 1b, 3a to 3b). The method may comprise one or more additional optional features corresponding to one or more aspects of the proposed concept or one or more examples described above or below.

Various examples of the present disclosure relate to a concept for providing explicit indicators of events, i.e., features of interest. The proposed concept is based on analyzing, in the background, the information available to the microscope, and using certain criteria to provide indicators for the current situation. These indicators may comprise indicators about available information which is not currently shown to the user. For example, when the user is in white light mode and there are fluorescence signals detected in one of the fluorescence imaging channels. These indicators may comprise indicators about available information, which is currently shown to the user, but there is the risk that the user might miss, such as weak fluorescence imaging signals which are shown as low contrast pseudocolor. These indicators may comprise warnings about events that the relevant information is not currently shown, or it is shown but the user might have missed, such as the presence of bleeding.

The proposed concept may check for specific events. If something happens, i.e., a feature of interest occurs, an explicit indicator such as a sign may be shown. For example, the indicators may be shown as symbols, as AR (Augmented Reality), e.g. a fluorescence imaging pseudocolor blinking periodically. Events/features of interest may be detected from a combined evaluation of multiple different data.

In the following, an example is shown, where the pseudocolor overlay is hard to perceive in the digital view. Fig. 3a shows a schematic drawing of an example of a combined white light and fluorescence digital view. A low-contrast pseudocolor overlay is overlaid over a tissue portion 310. However, due to the low intensity of the fluorescence emissions and the high brightness of the area in the white light image, the pseudocolor overlay is hard to perceive. In Fig. 3b, the same surgical site is shown in representation of the fluorescence imaging component of the imaging sensor data. Fig. 3b shows a schematic diagram of an example of a fluorescence digital view. In this view, the tissue portion 320 is easy to make out, as the fluorescence emissions are limited to the area of the tissue portion. An indicator 330 is overlaid over the fluorescence digital view, providing an outline of the tissue portion 320. The same outline may be added to the combined digital view to highlight the extent of the tissue portion in the combined digital view.

As a practical example, during tumor surgery, a surgeon may think that they are in the combined representation but might actually use the white light only-representation at the time. When turning back to the white light only-representation, after a tumor resection in the combined mode, the surgeon might not know whether they missed a part of the tumor. The proposed concept may address this issue by checking for specific events. If an event, i.e., a feature of interest, occurs, an explicit indicator may be shown, such as a sign. As a consequence, the surgeon may be informed on whether there are still fluorescence imaging signals, even in the white light only-representation. For example, a warning message may be be displayed if fluorescence is detected during a tumor resection and the surgeon is in white light only-representation. The proposed concept may be applied when the surgical microscope system is used in microsurgery, e.g., oncology surgery or vascular surgery. It may be implemented using software.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 3b. Alternatively, a microscope may be part of or connected to a system as described in connection with one or more of the Figs. 1 to 3b. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference Signs

- 100: Surgical microscope system
- 105: Base unit
- 110: System
- 112: Interface
- 114: Processor
- 116: Storage device
- 120: Microscope
- 122: Optical imaging sensor
- 130: Display device
- 130a: Ocular display
- 130b: Auxiliary display
- 140: Arm
- 210: Obtaining imaging sensor data
- 220: Generating a digital view
- 230: Detecting at least one feature of interest
- 240: Generating a visual indicator
- 250: Providing a display signal
- 310: Tissue
- 320: Feature of interest
- 330: Visual indicator
- 400: System
- 410: Microscope
- 420: Computer system

## Claims

1. A system (110; 420) for a microscope (120; 410) of a surgical microscope system (100; 400), the system (110) comprising one or more processors (114) and one or more storage devices (116), wherein the system is configured to:
obtain imaging sensor data from at least one optical imaging sensor (122) of the microscope, the imaging sensor data comprising a first component that is based on reflectance imaging and a second component that is based on fluorescence imaging;
generate a digital view of a surgical site based on at least the first component of the imaging sensor data;
detect at least one feature of interest in the second component of the imaging sensor data;
generate a visual indicator of the at least one feature of interest; and
provide a display signal to a display device (130) of the surgical microscope system, the display signal comprising the digital view and the visual indicator.

2. The system according to claim 1, wherein the system is configured to detect the at least one feature of interest based on fluorescence emissions emitted by the feature of interest.

3. The system according to one of the claims 1 or 2, wherein the system is configured to generate the visual indicator on the at least one feature of interest if the second component is omitted in the generation of the digital view.

4. The system according to one of the claims 1 to 3, wherein the system is configured to determine a visibility of the at least one feature of interest, and to generate a visual indicator for a feature of interest if the visibility of the feature of interest is deemed to fail a visibility criterion.

5. The system according to claim 4, wherein a feature of interest detected in the second component of the imaging sensor data is deemed to fail the visibility criterion if the second component is omitted in the generation of the digital view.

6. The system according to claim 4, wherein the system is configured to generate the digital view based on the first component and based on the second component of the imaging sensor data.

7. The system according to claim 6, wherein the system is configured to determine a feature detected in the second component to fail the visibility criterion if the size of the feature, relative to the digital view, is below a size threshold.

8. The system according to one of the claims 6 or 7, wherein the system is configured to determine a feature detected in the second component to fail the visibility criterion if a contrast or intensity of fluorescence imaging indicator highlighting the feature in the digital view relative to adjacent portions of the digital view is below a contrast threshold or intensity threshold, respectively.

9. The system according to one of the claims 6 to 8,wherein the at least one visibility criterion is based on at least one of a size of the one or more features of interest, a contrast of the one or more features of interest relative to the digital view, a visual intensity of the one or more features of interest in the digital view, and the presence of an indicator highlighting the one or more features of interest in the digital view.

10. The system according to one of the claims 6 to 9, wherein the system is configured to generate the digital view as a composite image with a pseudocolor representation of the second component that is combined with a color representation of the first component or as an image with a color representation of the first component and a monochrome representation of the second component that are shown separately.

11. The system according to one of the claims 1 to 10, wherein the system is configured to perform object detection based on the first and/or second component of the optical imaging data to determine whether a feature is a feature of interest.

12. The system according to one of the claims 1 to 11, wherein the visual indicator is one of a blinking indicator, an indicator having an increased contrast relative to adjacent portions of the digital view, an indicator drawing an outline around the feature of interest, and an indicator for prompting a surgeon to switch the digital view to show fluorescence emissions in isolation.

13. The system according to one of the claims 1 to 12, wherein the system is configured to include the visual indicator in the display signal as one of an overlay, an indicator included in a composite reflectance and fluorescence image, and a warning image.

14. A method for a microscope of a surgical microscope system, the method comprising:
obtaining (210) imaging sensor data from at least one optical imaging sensor of the microscope, the imaging sensor data comprising a first component that is based on reflectance imaging and a second component that is based on fluorescence imaging;
generating (220) a digital view of a surgical site based on at least the first component of the imaging sensor data;
detecting (230) at least one feature of interest in the second component of the imaging sensor data;
generating (240) a visual indicator of the at least one feature of interest; and
providing (250) a display signal to a display device of the surgical microscope system, the display signal comprising the digital view and the visual indicator.

15. A computer program with a program code for performing the method according to claim 14 when the computer program is executed on a processor.
